# EUROPEAN PATENT APPLICATION

(11) **EP 2 090 307 A1**
(43) Date of publication of application: **19.08.2009**
(21) Application number: 08101682.6
(22) Date of filing: 15.02.2008
(51) Int. Cl.: A61K 31/4168, A61K 31/726, A61K 31/728, A61K 9/00, A61P 19/02

(54) **Pharmaceutical composition for the treatment or prevention of osteoarticular diseases**

(71) Applicant: Bone Therapeutics, 6041 Gosselies (BE)
(72) Inventor: BASTIANELLI, Enrico, B-1640, RHODE-SAINT-GENESE (BE); ATTALI, Pierre, F-94300, VINCENNES (FR); VERVAET, Chris, B-8870, IZEGEM (BE)
(74) Representative: Van Malderen, Joëlle

(57) **Abstract**

The present invention is related to a pharmaceutical composition for the acute and/or chronic treatment (or prevention) of osteoarticular diseases, comprising
- possibly an adequate pharmaceutical carrier or diluent
- a polysaccharide and/or a glycosaminoglycan
- a compound activating the alpha 2 adrenergic receptor
- an anti-inflammatory agent and stem cells.

## Description

### Field of the invention

The present invention is in the pharmaceutical field and is related to a new pharmaceutical composition for treatment or prevention of the acute and/or chronic osteoarticular diseases.

### Background of the invention

Osteoarthritis, the most common form of arthritis, is a disease characterised by a slow degenerative processes in the articular cartilage, subchondral bone associated with marginal osteophyte formation, and low grade inflammation. Osteoarthritis is believed to affect 15% of the population in its chronic form. Of those, one-quarter are severely disabled. Most cases of osteoarthritis have no known cause and are referred to as primary osteoarthritis. When the cause of the osteoarthritis is known, the condition is referred to as secondary osteoarthritis. Secondary osteoarthritis is caused by another disease or condition. Conditions that can lead to secondary osteoarthritis include repeated trauma or surgery to the joint structures, abnormal joints at birth (congenital abnormalities), gout, diabetes, and other hormone disorders. Other forms of arthritis are systemic illnesses, such as rheumatoid arthritis and systemic lupus erythematosus (SLE).

Osteoarthritis involves mainly the hips, knees, spine, and the interphalangeal joints. The most common symptom of osteoarthritis is pain in the affected joint(s) after repetitive use. Joint pain is usually worse later in the day. There can be swelling, warmth, and creaking of the affected joints. Pain and stiffness of the joints can also occur after long periods of inactivity. In severe osteoarthritis, complete loss of cartilage cushion causes friction between bones, causing pain at rest or pain with limited motion.

Osteoarthritis is characterized by a slow degradation of cartilage over several years. In normal cartilage, a delicate balance exists between matrix synthesis and degradation; in osteoarthritis, however, cartilage degradation exceeds synthesis. The balance between synthesis and degradation is affected by age and is regulated by several factors produced by the synovium and chondrocytes, including cytokines, growth factors, aggrecanases, and matrix metalloproteinases In addition to water, the extracellular matrix is composed of proteoglycans, made up of glycosaminoglycans attached to a backbone made of hyaluronic acid, entrapped within a collagenous framework or fibrillary matrix. A significant proteoglycal in articular cartilage is aggrecan, which binds to hyaluronic acid and helps provide the compressibility and elasticity of cartilage. Aggrecan is cleaved by aggrecanases, leading to its degradation and to subsequent erosion of cartilage. The loss of aggrecan from the cartilage matrix is one of the first pathophysiological changes observed in OA.

Cytokines produced by the synovium and chondrocytes, especially IL-1beta and Tumor Necrosis Factor alpha (TNF-alpha), are also key players in the degradation of cartilage. IL-1beta is spontaneously released from cartilage of osteoarthritis but not normal cartilage. Both IL-1beta and TNF-alpha stimulate their own production and the production of other cytokines (e.g., IL-8, IL-6, and leukotriene inhibitory factor), proteases, and prostaglandin E₂ (PGE₂). Synthesis of the inflammatory cytokines IL-1beta and TNF-alpha and expression of their receptors are enhanced in osteoarthritis. Both cytokines have been shown to potently induce degradation of cartilage *in vitro.* Other proinflammatory cytokines overexpressed in osteoarthritis include IL-6, IL-8, IL-11, and IL-17, as well as leukotriene inhibitory factor.

The extracellular matrix (ECM) composing the cartilage is degraded by locally produced matrix metalloproteinases. Proinflammatory cytokines, including IL-1beta, TNF-alpha, IL-17, and IL-18, increase synthesis of matrix metalloproteinases, decrease matrix metalloproteinase enzyme inhibitors, and decrease extracellular matrix synthesis.

In an attempt to reverse the breakdown of the extracellular matrix, chondrocytes increase synthesis of matrix components including proteoglycans. Even though this activity increases, a net loss of proteoglycans in the upper cartilage layer is seen. Elevated anti-inflammatory cytokines found in the synovial fluid of osteoarthritis include IL-4, IL-10, and IL-13. Their role is to reduce production of IL-1beta, TNF-alpha, and matrix metalloproteinases, and inhibit prostaglandin release. Local production of growth and differentiation factors such as insulin-like growth factor 1, transforming growth factors, fibroblastic growth factors, and bone morphogenetic proteins also stimulate matrix synthesis.

### State of the Art

Currently available pharmacological therapies target palliation of pain and include analgesics (i.e. acetaminophen, cyclooxygenase-2-specific inhibitors, non-selective nonsteroidal anti-inflammatory drugs, tramadol, opioids). However, the clinical presentation of osteoarthritis is usually monoarticular or oligoarticular with fluctuations in intensity and localisation over time. It is therefore logical to consider local therapeutic modalities in order to avoid untoward systemic effects. Several compounds have been used intra-articularly (e.g., glucocorticoids, hyaluronic acid) or topically (e.g., capsaicin, methylsalicylate). However, the benefit of intra-articular glucocorticoides lasts only a few days (Barron, M. C., 2007, J. Am. Osteopath., 107, ES21-27).

Polysaccharides form a class of materials whose recognition of the potential utility is growing. Apart from their biological activity, one of the more important properties of polysaccharides in general is their ability to form hydrogels. Hydrogel formation can occur by a number of mechanisms and is strongly influenced by the types of monosaccharide involved, as well as the presence and nature of substituent groups. Polysaccharide gel formation is generally of two types: hydrogen bonded and ionic. Hydrogen-bonded gels are typical of molecules such as agarose (thermal gellation) and chitosan (pH-dependent gellation), whereas ionically bonded gels are characteristic of alginates and carrageenans.

Proteoglycans are one of the major macromolecules found in articular cartilage. These molecules consist of a core protein and covalently attached glycosaminoglycans (GAG) chains. The GAGs are long, unbranched heteropolysaccharides, consisting of repeated disaccharide units, with the general structure: Muronic acid amino sugar. The cartilage-specific GAGs include chondroitin 4-sulfate (glucuronic acid and N-acetylgalactosamine with an S0₄ on the 4-carbon position), chondroitin 6-sulfate (glucuronic acid and N-acetylgalactosamine with an SO₄ on the 6-carbon position) and keratan sulfate (galactose and N-acetyl-glucosamine with an SO₄ on the 6-carbon position).

These molecules are capable of forming hydrogel complexes with oppositely charged ionic polymers, particularly the cationic polysaccharide chitosan. This interaction may form the basis of a new materials approach to cartilage tissue engineering. The other important cartilage GAG is hyaluronic acid (glucuronic acid and N-acetyl-glucosamine). This molecule is one of the major components in synovial fluid. Hyaluronic acid molecules are also present in cartilage matrix as the backbone structure in proteoglycan aggregates. In general, hyaluronic acid plays a major role as an organizer of the extracellular matrix. Purified hyaluronic acid is employed as a structural biomaterial because of its high molecular weight and gel forming ability. The properties of the molecule may be broadly altered by chemical modification. For example, partial esterification of the carboxyl groups reduces the water solubility of the polymer and increases its viscosity. Extensive esterification generates materials that form water-insoluble films or swellable gels. Ethyl and benzyl esterified hyaluronate membranes have excellent healing responses and biodegradability properties. The fully esterified membranes have in vivo lifetimes of several months, whereas the partially esterified forms have been shown to degrade within a few weeks.

Hyaluronic acid is responsible for the viscoelastic quality of synovial fluid that acts as both a lubricant and shock absorber. In synovial fluid, hyaluronic acid coats the surface of the articular cartilage and shares space deeper in the cartilage among collagen fibrils and sulfated proteoglycans. It protects the cartilage and blocks the loss of proteoglycans from the cartilage matrix into the synovial space, maintaining the normal cartilage matrix. In synovial fluid from knee joints in osteoarthritis, concentrations of hyaluronic acid, glycosaminoglycans, and keratan sulfate are lower than in synovial fluid from normal knee joints. Additionally, experiments using rabbit synovial cells showed that the proinflammatory cytokines IL-1 and TNF-alpha stimulate the expression of hyaluronic acid synthetase, which may contribute to the fragmentation of hyaluronic acid under inflammatory conditions. Exogenous hyaluronic acid may facilitate the production of newly synthesized hyaluronic acid. Hyaluronic acid and derivatives have been used as therapeutic aids in the treatment of osteoarthritis as a means of improving lubrication of articulating surfaces and thus reducing joint pain. Several in vitro culture studies have also demonstrated that hyaluronic acid has a beneficial effect by inhibiting chondrocytic chondrolysis mediated by fibronectin fragment. Hyaluronic acid has also been shown to have anti-inflammatory effects, as well as inhibitory effects on prostaglandin synthesis, and proteoglycan release and degradation.

Chitosan, a partially de-acetylated derivative of chitin, found in arthropod exoskeletons is another proteoglycan, a linear polysaccharide consisting of beta (1-4) linked D-glucosamine residues with a variable number of randomly located N-acetyl-glucosamine groups. It thus shares some characteristics with various GAGs and hyaluronic acid present in articular cartilage, Depending on the source and preparation procedure, chitosan's average molecular weight may range from about 50 to about 1000 kDa. Commercially available preparations have degrees of deacetylation ranging from about 50 to about 90%. Chitosan is a semi-crystalline polymer and the degree of crystallinity is a function of the degree of deacetylation. Crystallinity is maximum for both chitin (i.e. 0% deacetylated) and fully deacetylated (i.e. 100%) chitosan. Minimum crystallinity is achieved at intermediate degrees of deacetylation.

Much of the potential of chitosan as a biomaterial stems from its cationic nature and high charge density. The charge density allows chitosan to form insoluble ionic complexes or complex coacervates with a wide variety of water-soluble anionic polymers.

In fact, chitosan oligosaccharides have been shown to have a stimulatory effect on macrophages, and the effect has been linked to the acetylated residues. Furthermore, both chitosan and its parent molecule, chitin, have been shown to exert chemoattractive effects on neutrophils in vitro and in vivo. In vivo, chitosan is degraded by enzymatic hydrolysis. The mechanical properties of chitosan scaffolds are mainly dependent on the pore sizes and pore orientations.

Hyaluronic acid, a glycosaminoglycan, is widely used for the treatment of osteoarthritis of the knee. A survey of 2 general practices in the United Kingdom showed that about 15% of patients with osteoarthritis received intra-articular treatment with hyaluronic acid preparations. Because of its viscoelastic quality, it may replace synovial fluid. Furthermore, it may reduce the perception of pain. Beneficial molecular and cellular effects have also been reported. Hyaluronic acid is frequently applied by intra-articular injection, but the evidence concerning its clinical relevance is conflicting. State-of-the-art systematic reviews and meta-analyses were published recently, and their authors concluded that intra-articular hyaluronic acid, at best, has a small effect, a clinically meaningful effect meaning an improvement of at least 15 mm on the visual analog scale of pain (Bellamy et al, 2006; Cochrane Database Syst Rev. 2006 Apr 19;(2):CD005321). These data form the basis for the use of intra-articular administration of hyaluronic acid in patients with osteoarthritis. The benefits are sometimes noticed only one year after injections and, in some experiments, injections must be performed three to five-times a week (Barron, M. C., 2007, J. Am. Osteopath., 107, ES21-27).

Alpha-2-adrenergic receptor ligands especially agonists are drugs commonly used in medical practice as antihypertensive substance and in clinical anesthesiology as component of general and locoregional anesthesia and analgesia. They produce anxiolysis, analgesia, sedation, anesthetic sparing effects and perioperative hemodynamic stabilizing effects. Negative neurotoxicity studies allow their use (mainly clonidine) by systemic and perimedullar routes and for peripheral nerve blocks. Among the clinically available alpha-2-adrenoreceptors agonists, clonidine remains widely used: the substance is devoid of neurotoxicity and displays less side effects (i.e. hypotension and sedation) than the more potent and also more alpha-2-adrenergic receptor selective agonist, dexmedetomidine. Clonidine, a potent alpha-2-adrenergic receptors partial agonist, was used primarily for the treatment of hypertension. This drug stimulates alpha-2-adrenergic receptors in the vasomotor centers, causing a reduction of sympathetic outflow from the central nervous system. Both cardiac output and peripheral resistance are reduced resulting in a decrease in blood pressure. Higher concentrations cause a vasoconstriction by activation of postsynaptic receptors in vascular smooth muscle. However, the significant advantages of the drug are counter balanced by side effects including dryness of the mouth, sedation and dizziness. Furthermore, other activities of these compounds such as anti-inflammatory effect have never been reported by oral administration.

Besides the well-known analgesic effects of spinally administered alpha-2-adrenergic (alpha-2-adrenoceptors) agonists, their peripheral use has been commonly reported in acute pain conditions. For perioperative analgesic techniques, clonidine is added to local anesthetic in peritroncular nerve blocks to enhance potency and duration of analgesia. ZOUHER A et al. ; Paediatr Anaesth. 2005 Nov;l5(11):964-70; Luiz-Cleber P. et al. ; Anesth Analg. 2005 Sep;101(3):807-11, Murphy DR "A non-surgical approach to low back pain" Med. Health R. I., 2000 April; 83(4): 104-7). Further, intra-articular injection of clonidine and its adjunction to local anesthetic solution for intravenous regional anesthesia have also displayed anti-nociceptive effect S. Armand et al; Br J Anaesth. 1998 Aug; 81(2):126-34; Gentili M, et al. Pain 1996; 64: 593-596; Reuben S, et al. Anesthesiol. 1999; 91: 654-658). However, the effect is here designed not to last for days or weeks.

Alpha-2-adrenoceptors agonists are known to block the tissue content increase of pro-inflammatory cytokines, such as TNF-alpha and ILlbeta and increase the tissue content of anti-inflammatory cytokine TGF beta. This has been shown in inflammatory neuropathic pain model by partial ligation of sciatic nerve, by applying locally alpha 2 adrenergic receptor agonists by peripheral nerve block.

### Summary of the invention

The invention is related to a intra-articular pharmaceutical composition for a treatment and/or a prevention of acute or chronic osteoarticular diseases and acute or chronic osteoarticular symptoms especially osteoarthritis, this composition comprising
- possibly an adequate pharmaceutical carrier or diluent
- a polysaccharide and/or a glycosaminoglycan
- a compound activating the alpha-2-adrenergic receptor
- possibly a sufficient amount of an anti-inflammatory agent (compound), preferably selected from the group consisting of a steroidal (such as prednisolone, dexamethasone, betamethasone, triamcinolone...), a non-steroidal anti-inflammatory compound (such as ibuprofen, diclofenac, naproxen, etc...), a disease modifying antirheumatic drug (such as methotrexate, leflunoimde etc...), an anti-CD20 agent, anti-cytokine agent (such as anti-IL1, anti-IL6, anti IL-17) an anti-TNF agent (such as infliximab, etanercerpt, adalimumab, rituximab, etc) or a mixture thereof, and,
- possibly stem cells (differentiated or not), preferably human mesenchymal stem cells adult or (non human) embryonic stem cells having potential anti-inflammatory properties.

In the pharmaceutical composition of the invention the polysaccharide or the glycosaminoglycan are present as a matrix preferably in the form of a paste or a gel, more preferably an hydrogel with a sufficient amount of an aqueous solvent.

Advantageously, the compound interacting with the alpha-2-adrenergic receptor is an alpha-2-adrenergic receptor agonist.

In the composition according to the invention the polysaccharide-based hydrogel and the glycosaminoglycan are not covalently linked.

Preferably, the glycosaminoglycan is selected from the group consisting of hyaluronic acid with low (< 900 kDa) or high (> 900 kDa) molecular mass.

In the composition of the invention, the hyaluronic acid and the alpha 2 adrenergic receptor agonist are not covalently linked or are covalently linked.

In the composition according to the invention the polysaccharide-based hydrogel or matrix or the glycosaminoglycan are selected from the group consisting of proteoglycan, chondroitin sulfate, keratin sulphate, hyaluronic acid (including their derivatives) chitosan, a chitosan or chitin derivative, or a mixture thereof.

The alpha-2-adrenergic receptor agonist is selected from the group consisting of clonidine, p-aminoclionidine, tiamenidine, 5-bromo-6-(2 imidazolidine-2-ylamino) quinoxaline, dexmedetomidine, detomidine, medetomidine, alphamethyldopa, oxymetazonline, brimonidine, tizanidine, mivazerol, lofexidine, xylazine, guanabenz, guanfacine, guanclofine, guanoxabenz, or a derivative or structural analogue thereof, alpha-methyinorepherine, azepexole, indoramin, 6-allyl-2-amino-5,6,7,8-tetrahydro4H-thiazolo [4,5-d]azepine diHCl.

The inventors have discovered that the pharmaceutical composition according to the invention is suitable for a treatment or prevention of acute and chronic osteoarticular diseases and associated symptoms (especially osteoarticular pain, mobility or function), of inflammatory origin, such as osteoarthritis, degenerative arthristis, degenerative arthritis, gonarthrosis, coxarthrosis, and other inflammatory general conditions in which joints are involved, such as autoimmune diseases, especially rheumatoid arthritis and systemic lupus erythematosus (SLE) spondyloarthropathies, polymyalgia rheumatica, ankylosing spondylitis, Reiter's Syndrome, psoriatic arthropathy, enteropathic arthritis (related to inflammatory bowel disease, such as haemorrhagic colitis and Crohn's disease), neuropathic arthropathy, acute rheumatic fever, gout, chondrocalcinosis, calcium hydroxyapatite crystal deposition disease, Lyme disease and all other degenerative joint diseases.

Another aspect of the present invention is related to the use of the pharmaceutical composition for the manufacture of a medicament in the treatment and/or the prevention of these mentioned diseases and/or symptoms (pain) induced by these diseases, possibly combined with an anti-inflammatory effect useful for the treatment and/or the prevention of diseases or symptoms of inflammatory origin.

According to a preferred embodiment of the present invention the composition presents a preferred formulation, preferably being an injectable solution for delivering an efficient amount (therapeutic dose) of the active compound present in the composition to a mammal subject including a human patient, especially in the knees, the hips and the spine of the subject.

Preferably, this injectable solution comprises from about 0.1 mg to about 100 mg/kg preferably from about 1 mg to about 10 mg/kg of body weight of this polysaccharide and/or glycosaminoglycan, preferably in the form of a polysaccharide-based hydrogel and may comprise from about 0.1 mg to about 100 mg/kg of patient body weight preferably from about 0.1 mg to about 0.8 mg/kg of patient body weight of an anti-inflammatory compound being a compound activity the alpha-2-adrenergic receptor, preferably an alpha-2-adrenergic receptor agonist.

The injectable solution is adequate for intra-articular administration (percutaneous injection) in a joint of a mammal subject, preferably of a human patient.

This formulation is also adequate for local administration (percutaneous injection in/or in the vicinity of an inflamed joint of a mammal subject, preferably of a human patient), local administration injection that does concern the epidermis, the muscle or any deep organs.

The present invention is related to the surprising discovery that this intraarticular administration to a mammal subject, particularly a human patient of this pharmaceutical composition results in an improvement of symptoms associated with osteoarticular diseases such as a relief of osteoarticular pain, an improvement of joint mobility and/or function, a decrease in articular accumulation of inflammatory liquid, a shortening of the time to onset of the therapeutic activity of the first component, an increase in the duration of action of the first component and a reduction of joint degeneration or its progress possibly induced by the above mentioned diseases or pathologies.

The invention is also directed to a method for a treatment and/or a prevention (prophylaxis) of a condition selected from the group consisting of the above mentioned diseases or symptoms through an administration to a mammal subject, preferably a human patient of a sufficient amount of the pharmaceutical composition of the invention, through an intra-articular administration, particularly in the knees, the hips and the spine of the mammal subject; preferably the human patient.

A combined intra-articular administration of hyaluronic acid and an alpha 2 adrenergic receptor agonist allows for a shorter onset of action, a longer duration of action, for a superior or at least equivalent efficacy and/or a possible decrease of side-effects associated with hyaluronic acid and with administration of alpha 2 adrenergic receptor agonists.

The composition and method of the present invention also allows a decrease of the number of required administrations to obtain a desired efficacy and allows for a faster onset of action.

A last aspect of the present invention is related to a kit of parts comprising one or more vials with the elements (carrier/diluent, polysaccharide, glycosaminoglycan, 2-2-adrenergic receptor compound, anti-inflammatory agent, stem cells or a combination thereof) of the composition of the invention and a device for delivering this composition to an inflamed joint of a mammal subject preferably a human patient suffering from the above mentioned osteoarticular diseases or symptoms (pain) and having
- reservoir means for storing this pharmaceutical composition,
- piston means movable along the longitudinal axis of the reservoir for dispensing this pharmaceutical composition and,
- a hollow needle mounted on said reservoir means for delivering this pharmaceutical composition to the peripheral nerve of the mammal subject.

These and other objects and features of the invention will become more fully apparent when the following detailed description of the invention is read in conjunction with the accompanying examples and figures.

### Brief description of the figures and tables

FIG. 1 shows the VAS scale assessing pain from 0 "no pain at all" to 100 "maximum pain" after 3 intraarticular administration of clonidine 150 µg and hyaluronic acid at day 0, day 7 and day 14 in 2 patients suffering from gonarthritis.
FIG. 2 shows the WOMAC score assessing pain and function of the knee after 3 intra-articular administration of clonidine 150 µg and hyaluronic acid at day 0, day 7 and day 14 in 2 patients suffering from gonarthritis (worse situation = 96).
FIG. 3 shows the Lequesne score assessing algofunctional score of the knee after 3 intra-articular administration of clonidine 150 µg and hyaluronic acid at day 0, day 7 and day 14 in 2 patients suffering from gonarthrosis (worse situation = 24).
FIG 4 shows the VAS scale assessing pain from 0 "no pain at all" to 100 "maximum pain" after one intra-articular administration of clonidine 150 µg and hyaluronic acid at day 0 in 1 patient suffering from coxarthrosis.
FIG 5 shows the WOMAC score assessing pain and function of the hip after one intraarticular administration of clonidine 150 µg and hyaluronic acid at day 0 in 1 patient suffering from coxarthrosis.
FIG 6 shows the Lequesne score assessing pain and function of the hip after one intra-articular administration of clonidine 150 µg and hyaluronic acid at day 0 in 1 patient suffering from coxarthrosis.

### Detailed description of the invention

The term "intra-articular" as used herein refers to a percutaneous injection of a joint with the pharmaceutical composition of the invention.

The term "local administration" as used herein refers to a percutaneous injection in or in the vicinity of an inflamed joint. Local administration injection thus concerns the epidermis, the dermis, the muscle, or any deep organ.

The main advantages to local administration are to selectively restrict the analgesic effect to the injured areas. Furthermore, the local administration allows for high local concentration levels with little or no systemic release. The local administration and the intra-articular injection of hyaluronic acid is a recognised treatment of the above mentioned conditions.

The local administration and the intra-articular injection of alpha 2 adrenergic agonists are easily realizable and provide long lasting effect in combination with proteoglycans. In consequence, the problems related to placement of an invasive drug delivery system and the problems of bothersome side-effects, due to systemic release, can be strongly minimized. Health-related quality of life, patient satisfaction and economic assessment might be improved with such a treatment, especially in chronic conditions.

Preferably, the administrated compound is selected from the group consisting of a polysaccharide-based hydrogel or a glycosaminoglycan, for example hyaluronic acid or a salt thereof or an ester of hyaluronic acid with an alcohol of the aliphatic, heterocyclic or cycloaliphatic series, or a sulphated form of hyaluronic acid or combination of agents containing hyaluronic acid. Suitable dosages of a polysaccharide-based hydrogel or a glycosaminoglycan or a derivative thereof will typically be from about 0.1 mg to about 100 mg /kg body weight per day or from about 0.5 mg to about 10 mg/kg body weight per day more preferably from about 2 mg to about 8 mg by body weight per day and suitable dosage amounts for alpha 2 adrenergic receptor agonists.

In a preferred embodiment, the alpha 2 adrenergic receptor agonist may be clonidine, p-aminoclionidine, tiamenidine, 5-bromo-6-(2 imidazolidine-2-ylamino) quinoxaline, dexmedetomidine, detomidine, medetomidine, alphamethyldopa, oxymetazonline, brimonidine, tizanidine, mivazerol, lofexidine, xylazine, guanabenz, guanfacine, guanclofine, guanoxabenz, or a derivative or structural analogue thereof, alpha-methyinorepherine, azepexole, indoramin, 6-allyl-2-amino-5,6,7,8-tetrahydro4H-thiazolo [4,5-d]azepine diHCl and analogs thereof.

The active compounds used in accordance with the invention are known. Pharmaceutical preparations containing hyaluronic acid are commercially available as are clonidine and other alpha 2 adrenergic receptor agonists. The compounds can be manufactured in a known manner essentially in accordance with the processes described in the prior art.

The term "clonidine" as used herein refers to N-(2,6-dichlorophenyl)-4,5-dihydro-1H-imidazol-2-amine and includes the pharmaceutically acceptable salts thereof, e.g., salts with inorganic acids, such as hydrohalic acids, or with organic acids, for example lower aliphatic monocarboxylic or dicarboxylic acids such as acetic acid, fumaric acid or tartaric acid or aromatic carboxylic acids such as salicylic acid are also suitable.

Clonidine is employed in a therapeutically effective amount. The present invention also encompasses the use of alpha 2 adrenergic receptor agonist for the manufacture of injectable formulations for a delivery to a joint or a close region thereof, a therapeutic dose of said agonist by intra-articular injection, wherein said solution comprises from about 3 µg to about 1500 µg of said alpha 2 adrenergic receptor agonist. Preferably, said formulation comprises from about 30 µg to about 500µg of said alpha 2 adrenergic receptor agonist. More preferably said solution comprises from 50 µg to 350 µg of said alpha 2 adrenergic receptor agonist. In a preferred embodiment, the alpha 2 adrenergic receptor agonist is clonidine. The actual concentration of clonidine may vary, depending on the nature and degree of the pain syndromes being treated and whether the drug is being administered for therapeutic or prophylactic purposes.

It is hypothesized that based on their anti-inflammatory properties, inhibition of pro-inflammatory cytokines TNF-alpha and IL1-beta and the increase of anti-inflammatory cytokines such as TGF beta, alpha 2 receptor agonists in injectable administration will have applications in osteoarticular inflammatory conditions and in diseases where there are osteoarticular inflammatory conditions, such as those above mentioned.

According to the method and the formulation of the invention, an injection of the combination of hyaluronic acid and an alpha 2 adrenergic receptor agonist form induces a long lasting pain relief, both in human case and in an animal model of osteoarticular pain. The method is safe, devoid of major drug's side effects and allows for acute as well as chronic treatment and or prophylaxis without the use of too invasive technique.

Preservatives may be incorporated in an amount effective for inhibiting growth of microbes, such as bacteria, yeast and molds, in the composition. Any conventional preservative against microbial contamination of the product can be employed so long as it is pharmaceutically acceptable and is unreactive with clonidine. Preferred preservatives are antimicrobial aromatic alcohols, such as benzyl alcohol, phenoxyethanol, phenethyl alcohol, and the like, and esters of parahydroxybenzoic acid commonly referred to as paraben compounds, such as methyl, ethyl, propyl, and butyl esters of parahydroxybenzoic acid and the like and mixtures thereof, but are not limited thereto. Particularly preferred are benzyl alcohol and phenoxyethanol.

Optionally, anaesthetic agent, such as lidocaine and the like, can be included. For administration according to the invention the active quantities of the compounds that alleviate neuropathic pain can be contained together with customary pharmaceutical excipients and/or additives in solid or liquid pharmaceutical formulations.

Liquid preparations such as solutions, suspensions or emulsions of the active ingredients, prepared by dissolving or dispersing the compounds, can contain the usual diluents such as water, oil and/or suspending aids such as polyethylene glycols and optional pharmaceutical adjuvants, in a carrier, such as, for example, aqueous saline, aqueous dextrose, glycerol, or ethanol, to form a solution or suspension and such like. Further additives such as preservatives, flavouring agents and such like may also be added.

### Examples

Four patients suffering from gonarthritis with knee effusion were administered one intra-articular injection of about 20 mg of hyaluronic acid and about 150 µg of clonidine in the injured knee. A second and a third intra-articular injection was realized 7 and 14 days later. Before each injection, the intra-articular liquid was removed and measured. Pain and mobility were evaluated at baseline (day 0), 48-72 hours after the intra-articular injections, before the next injection (day 7 and day 14) and at day 21. The evaluations consisted in a 0-100mm visual analog scale filled in by the patient recording pain intensity (0= no pain, 100= intolerable pain), the WOMAC score (worse possible score = 96) recording pain and mobility and an algofunctional scale (Lequesne Index, worse score = 24).

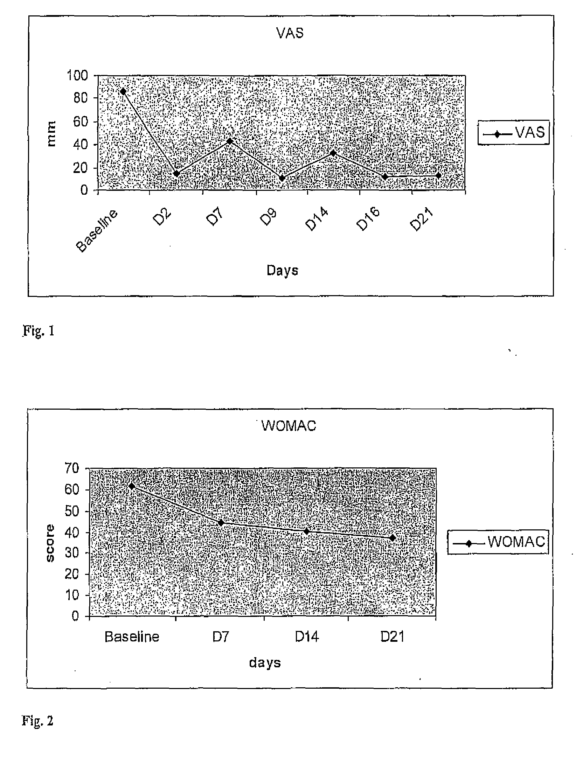

As shown in figures 1, 2 and 3, the intra-articular administration of the combination induced unexpectedly an relief in pain (decrease in VAS from 86.5 mm to 14.5 mm) in 48 hours and the 2 readministrations induced further improvement (VAS score = 11 at day 9, 12 at day 16). This unexpected effect by comparison to the effect of either product was maintained for at least 1 week after the 3^{rd} injection as VAS score was 13 at day 21. Same results were obtained using the WOMAC score which additionally evaluated mobility of the injured knee. The score decreased from 61 at baseline to 44, 41 and 37 at days 7, 14 and 21. Likewise the algofunctional score evaluated using the Lequesne scale showed a dramatic improvement with scores decreasing from 20 at baseline to 15, 13, 12 respectively at day 7, 14 and 21. These results were totally unexpected as hyaluronic acid has not been shown to decrease pain score by more than 15 mm in the metaanalyses that reassessed its efficacy and the effects of hyaluronic acid do not occur before 3 to 4 weeks. No data are available for clonidine in osteoarthrosis.

Two patients suffering from coxarthritis was administered one single intra-articular injection of about 20 mg of hyaluronic acid and about 150 µg of clonidine in the injured hip. Pain and mobility were evaluated at baseline (day 0), and at day 7, day 14, day 21 and day 28. The evaluations consisted in a 0-100mm visual analog scale filled in by the patient recording pain intensity (0= no pain, 100= intolerable pain), the WOMAC score (worse possible score = 96) recording pain and mobility and an algofunctional scale (Lequesne, worse score = 24).

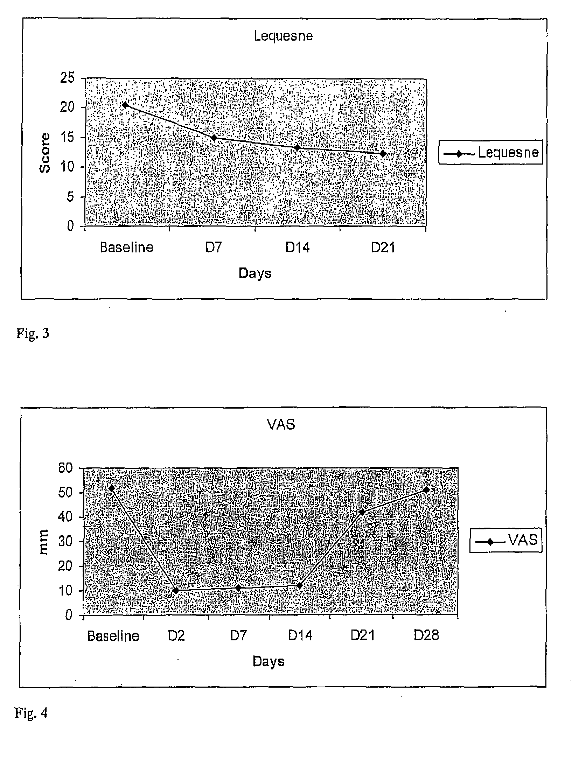

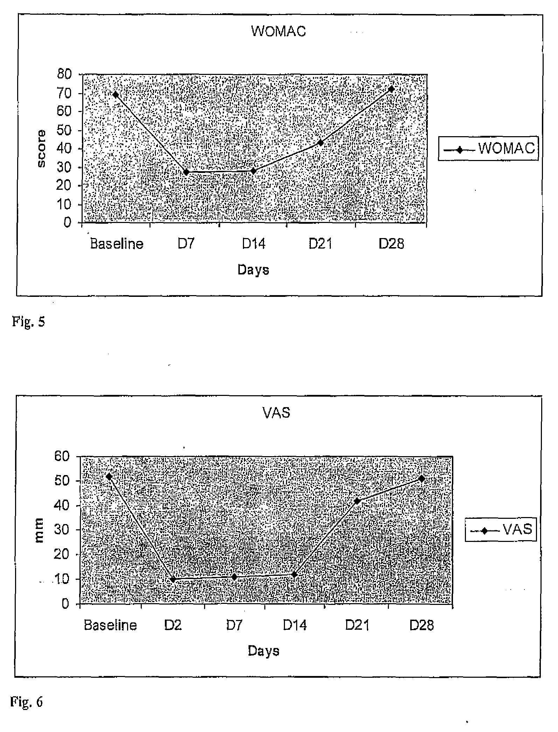

In patients with coxarthritis who have been treated with this combination, the effect of one intra-articular injection of about 20 mg of hyaluronic acid and about 150 µg of clonidine induced a immediate, marked, and prolonged effect as shown in fig 4, 5, and 6. VAS decreased from 52 mm at baseline to 10, 11, 42 and 51 at days 7, 14, 21 and 28. Likewise, the WOMAC score decreased from 69 at baseline to 27, 28, 43 and 72 at days 7, 14, 21 and 28 respectively and the algofunctional score from 13 to 8 and 8 at days 7 and 14 and returned to pre injection values at days 21 and 28: (13 and 14 respectively).

These results were unexpected as there is no reports of such improvement in the 3 weeks following the administration of hyaluronic acid. No data are available with clonidine. The prolonged antalgic effect was also totally unexpected.

## Claims

1. A pharmaceutical composition, comprising
- possibly an adequate pharmaceutical carrier or diluent
- a polysaccharide and/or a glycosaminoglycan
- an effective amount of a compound activating the alpha 2 adrenergic receptor, preferably an alpha 2 adrenergic receptor agonist
for a treatment and/or a prevention of acute or chronic osteoarticular diseases and/or symptoms by intra-articular injection.

2. The composition of claim 1, wherein the acute or chronic osteoarticular symptom is pain mobility and/or function.

3. The pharmaceutical composition according to the claim 1 or 2, wherein the alpha 2 adrenergic receptor agonist is selected from the group consisting of clonidine, p-aminoclionidine, tiamenidine, 5-bromo-6-(2 imidazolidine-2-ylamino) quinoxaline, dexmedetomidine, detomidine, medetomidine, alphamethyldopa, oxymetazonline, brimonidine, tizanidine, mivazerol, lofexidine, xylazine, guanabenz, guanfacine, guanclofine, guanoxabenz, or a derivative or structural analogue thereof, alpha-methyinorepherine, azepexole, indoramin, 6-allyl-2-amino-5,6,7,8-tetrahydro4H-thiazolo [4,5-d]azepine diHCl.

4. The pharmaceutical composition according to the claims 1 to 3, wherein the polysaccharide is present as a polysaccharide based hydrogel and wherein this polysaccharide based hydrogel and the glycosaminoglycan and the anti-inflammatory agent are or are not covalently linked.

5. The pharmaceutical composition according to the claims 1 to 4, wherein the polysaccharide or the glycosaminoglycan is selected from the group consisting of proteoglycan, a chondroitin sulphate, a keratin sulphate or a hyaluronic acid or its derivative, a chitosan, a chitosan or chitin derivative or a mixture thereof.

6. The pharmaceutical composition according to any of the preceding claims 1 to 5 which is an injectable solution.

7. The pharmaceutical composition according to any of the preceding claims, which comprises from 0.1 to 100 mg/kg of body weight of polysaccharide or glycosaminoglycan and from 0.1 µg to 1 mg of compound.

8. The pharmaceutical composition according to anyone of the preceding claims 1 to 7, wherein the osteoarticular disease, (particularly of inflammatory origin) is selected from the group consisting of osteoarthritis, degenerative arthritis, gonarthrosis, coxarthrosis, and other inflammatory general conditions or symptoms in which joints are involved, such as systemic lupus erythematosus, spondyloarthropathies, polymyalgia rheumatica, ankylosing spondylitis, Reiter's Syndrome, psoriatic arthropathy, enteropathic arthritis (related to inflammatory bowel disease such as haemorrhagic colitis and Crohn's disease) rheumatoid arthritis, neuropathic arthropathy, acute rheumatic fever, gout, chondrocalcinosis, calcium hydroxyapatite crystal deposition disease, and Lyme disease.

9. The pharmaceutical composition of any of the preceding claims 1 to 8, wherein the injection is done twice a week, preferably once a week, more preferably once in two or three weeks.

10. The pharmaceutical composition of any of the preceding claims 1 to 9 further containing another anti-inflammatory compound.

11. The pharmaceutical composition of any of the preceding claims 1 to 9 further containing human adult mesenchymal stem cells or non human embryonic mesenchymal stem cells.

12. A composition comprising
- possibly an adequate pharmaceutical carrier or diluent
- 0.1 to 100 mg/ kg of body weight of a polysaccharide and/or a glycosaminoglycan
- 0.1 µg to 1 mg of a compound activating the alpha 2 adrenergic receptor.

13. A kit of parts comprising
- a vial with the composition according to any of the preceding claims 1 to 12,
- a device for delivering the said composition to an inflamed joint of a mammal subject and having
- reservoir means for storing the said composition,
- piston means movable along the longitudinal axis of the reservoir for dispensing the said pharmaceutical composition,
- and an hollow needle mounted on said reservoir means for delivering the said pharmaceutical composition to the peripheral nerve of the mammal subject.
